(19) **Europäisches Patentamt**
**European Patent Office**
**Office européen des brevets**

(11) **EP 1 694 129 B1**

(12) **EUROPÄISCHE PATENTSCHRIFT**

(45) Veröffentlichungstag und Bekanntmachung des Hinweises auf die Patenterteilung:
**06.10.2010 Patentblatt 2010/40**

(51) Int Cl.:
*A22B 5/00* *(2006.01)*       *G01N 33/12* *(2006.01)*

(21) Anmeldenummer: **04797409.2**

(22) Anmeldetag: **06.11.2004**

(86) Internationale Anmeldenummer:
**PCT/DE2004/002458**

(87) Internationale Veröffentlichungsnummer:
**WO 2005/055728 (23.06.2005 Gazette 2005/25)**

(54) **VERFAHREN ZUM ERMITTELN DER QUALITÄT UND QUANTITÄTEN EINES SCHLACHTTIERKÖRPERS**

METHOD FOR DETERMINING THE QUALITY OF A SLAUGHTERED ANIMAL CARCASS AND QUANTITIES THEREIN

PROCEDE DE DETERMINATION DE LA QUALITE D'UNE CARCASSE D'ANIMAL ABATTU ET DES QUANTITES COMPRISES

(84) Benannte Vertragsstaaten:
**AT BE BG CH CY CZ DE DK EE ES FI FR GB GR HU IE IS IT LI LU MC NL PL PT RO SE SI SK TR**
Benannte Erstreckungsstaaten:
**LT LV**

(30) Priorität: **13.12.2003 DE 10358487**

(43) Veröffentlichungstag der Anmeldung:
**30.08.2006 Patentblatt 2006/35**

(73) Patentinhaber: **CSB-System AG**
**52511 Geilenkirchen (DE)**

(72) Erfinder: **SCHIMITZEK, Peter**
**52511 Geilenkirchen (DE)**

(74) Vertreter: **Haussingen, Peter et al**
**Dr. Weihrauch & Haussingen**
**Patent- und Rechtsanwälte**
**Göpenstrasse 37**
**06526 Sangerhausen (DE)**

(56) Entgegenhaltungen:
**EP-A- 0 649 282        DE-A1- 4 109 345**
**DE-A1- 10 050 836      DE-A1- 19 847 232**
**DE-A1- 19 952 628      DE-C1- 19 837 806**
**DE-C1- 19 936 032**

**Beschreibung**

Die Erfindung bezeichnet ein Verfahren zur nichtinvasiven Bestimmung der Handelsklasse, des Handelswerts, dem Marktwert und der Qualität eines Schlachttierkörpers auf der Basis optischer Bildverarbeitung, das vorzugsweise in Schlacht- und Fleischverarbeitungsbetrieben eingesetzt werden kann. Üblicherweise werden größere Schlachttiere, wie Schweine, entlang der Wirbelsäule gespalten und an Haken hängend mittels spezieller Transportsysteme zwischen verschiedenen Stationen befördert. An festgelegten Orten werden die jeweiligen Hälften der Schlachtschweine registriert, gewogen und bewertet.

Im Rahmen der Bewertung erfolgt eine Einstufung des Schweineschlachtkörpers in gesetzliche Handelsklassen auf der Grundlage des Muskelfleischanteils. Zur Ermittlung des Muskelfleischanteils sind mehrere Verfahren zulässig, bei denen eine Speckdicke (S) und ein Fleischmaß (F), jeweils gemessen in Millimetern, zueinander in Relation gesetzt und der Muskelfleischanteil (MF%) mittels einer amtlich festgelegten Schätzformel berechnet wird.

Die Werte für die Terme S und F können bei einem möglichen Verfahren an einem Kotelettstück sieben Zentimeter seitlich einer Trennlinie auf Höhe der 2./3. Rippe gemessen werden. In einem anderen üblichen Verfahren, dem Zweipunkte (ZP) - Verfahren, werden bei der durch Spaltung des Schlachtkörpers längs der Wirbelsäule hergerichteten Schweinehälfte das Speckmaß (S) an der dünnsten Stelle des Specks über dem Musculus Glutaeus Medius (MGM) und das Fleischmaß (F) als Stärke des Lendenmuskels, gemessen als kürzeste Verbindung des vorderen (cranialen) Ende des MGM zur oberen (dorsalen) Kante des Wirbelkanals, bestimmt.

Der Muskelfleischanteil (MF%) wird rechnerisch durch Einsetzen von (S) und (F) in die amtliche Formel

$$MF\% = 47,978 + \left(26,0429 * S / F\right) + \left(4,5154 * \sqrt{F}\right) - \left(2,5018 * \lg S\right) - \left(8,4212 * \sqrt{S}\right)$$

spezifisch für Deutschland ermittelt, mit dessen Wert die Handelsklasseneinstufung entsprechend der relevanten Verordnungen erfolgt.

Das Bestimmen der Messwerte entsprechend dem ZP-Verfahren kann sowohl manuell als auch automatisch erfolgen. Aus dem Stand der Technik sind eine Reihe von Dokumenten bekannt, die hierzu automatisch arbeitende Lösungen unter Einbeziehung optischer Bildverarbeitung beschreiben.

In den Druckschriften DD 298 310 A5 / DE 41 31 556 C2 sowie DE 41 09 345 C2 werden Verfahren zur Bestimmung bzw. Analyse von Schlachttierkörperhälften durch Bildverarbeitung beschrieben, bei denen die äußere Kontur, Speckschicht, Fleisch- und Rückenspeckverhältnis ermittelt werden, indem Aufnahmen von der Schlachttierhälfte mit Wirbelsäule und allen Zwischenwirbelschichten angefertigt werden. Als Fixpunkt zur Ermittlung der Parameter für die Zerlegung und Klassifizierung wird vom Kreuzbein der Wirbelsäule ausgegangen, das ebenso wie die anderen Wirbel mittels Objektanalyse bestimmt wird. Nachteilig an diesem Verfahren ist zum einen der hohe rechentechnische Aufwand zur Objektanalyse mit vordefinierten Kontur- und Objektparametern, andererseits läßt sich das Kreuzbein als Fixpunkt bei auftretenden Spaltfehlern im realen Verarbeitungsprozeß nicht immer hinreichend sicher selektieren.

Die Druckschrift DE 197 33 216 C1 beschreibt ein Verfahren zur Bewertung von Schlachttierhälften durch optische Bildverarbeitung, das in Anlehnung an die klassische Zweipunkt-Methode eine Klassifizierung anhand einer optischen Bildauswertung der erweiterten Lendenregion unter Ausschluß subjektiver Fehlerquellen ermöglicht. Die Schätzgenauigkeit zur Bewertung und somit zur Klassifizierung wurde gegenüber den bisher bekannten Bewertungsverfahren damit nicht wesentlich verbessert.

Es ist auch ein Verfahren zur Bewertung von Schlachttierhälften durch optische Bildverarbeitung aus der Druckschrift DE 198 47 232 C2 bekannt, bei dem ein photogrammetrisches Verfahren als Nachbildung der herkömmlichen Zweipunkt-Methode zur Bewertung eingesetzt wird. In der Lenden- und Schinkenregion werden zwei markante Punkte, von denen der erste das körperseitige Ende des Schloßknochens ist, der zweite das körperseitige Ende des MGM (Musculus Glutaeus Medium) darstellt und eine Gerade mit der Richtung des mittleren Verlaufs des Rückenspecks, photogrammetrisch erfaßt. Zur eigentlichen Bewertung werden die Längen von Teilstrecken genutzt, die sich auf einer Senkrechten auf der zum Schloßknochen parallel verschobenen Gerade in Höhe des zweiten markanten Punktes durch die Dicke des Rückenspecks ergeben. Bei diesem Verfahren werden zwar die subjektiven Messfehler der manuell durchgeführten ZP-Methode ausgeschlossen, die Schätzgenauigkeit zur Bewertung wird jedoch auch damit nicht wesentlich erhöht.

Aus der Druckschrift DE 199 36 032 C1 ist ein weiteres Verfahren bekannt, das mittels optischer Bildverarbeitung eine automatische Qualitätsbeurteilung von Schlachttierhälften, insbesondere von Schlachtschweinen gewährleistet, wobei gegenüber den bekannten Verfahren eine höhere, reproduzierbare Schätzgenauigkeit erzielt wird, die durch Fehler im Spaltprozeß des Schlachttiers nur unwesentlich, durch nicht absolut senkrechte Bilderfassung zur Spaltebene nicht beeinflußt werden kann, wobei eine optische Aufnahme der Schlachttierhälfte in der Spaltebene, im Bereich der Schinken- und Lendenregion photogrammetrisch auf der Grundlage bestimmter markanter Bezugspunkte ausgewertet wird.

Als markante Bezugspunkte werden dabei die Wirbelsäule, der Schloßknochen, die dünnste Speckdicke am MGM und die Konturen des Rückenspecks im ausgewählten Bereich genutzt. Der zur Beurteilung der Qualität maßgebliche Magerfleischanteil wird durch Addition von zueinander ins Verhältnis gesetzter, zum geraden Verlauf des Rückenmarkkanals senkrechter Teilstrecken im Bereich des Fleischs und der Speckschicht unter Einbeziehung von aus Regressionsberechnungen ermittelter Konstanten für jeden Term und einer Grundkonstante berechnet.

Im Rahmen des Verfahrens wird zwar der Messwert für das Speckmaß (S) in Ubereinstimmung mit den gesetzlichen Vorschriften an richtigen Punkten ermittelt, jedoch wird das Fleischmaß (F) nicht bestimmt, wodurch die Berechnung des Muskelfleischanteils (MF%) nicht mit der amtlichen Formel erfolgt, somit eine Einstufung in die Handelsklassen nicht erfolgen kann.

Ein Verfahren zur Handelewertbestimmung der Teilstücke von Schweineschlachttierkörpern ist aus der Druckschrift DE 199 52 628 A1 bekannt, wobei Gewichte, Gewichts- und Fleischanteile von Teilstücken wie Schinken, Kotelett, ausgelöstes Kotelett, Filet, Schulter, Bug, Bauch und/oder weiterer selbständig handelbarer oder weiterzuverarbeitender Teilstücke mittels Online - Bewertung von Schweinehälften bestimmt wird. Zur Durchführung des Verfahrens werden den Körperbau beschreibende Prädiktoren bestimmt, die aus dem äußeren Konturverlauf der Schweinehälfte und der daraus abzuleitenden Fläche, Lage und Verlauf der Wirbelsäule und daraus abzuleitender Längen und Flächen von Teilbereichen des Schlachttierkörpers sowie einer für die Schweinehälften gewonnenen, durch die relative Dicke und den Verlauf der näherungsweise gesamten subkutanen Speckschicht des Rückenbereichs repräsentierende Verfettungsinformationen resultieren. Die Prädiktoren werden zueinander unter Berücksichtigung zwischen ihnen bestehender statistischer Zusammenhänge in Relation gesetzt, wodurch das Gewicht von interessierenden Teilstücken, deren Gewichts- und Fleischanteile am Gesamtgewicht der Karkasse online in der Schlachtlinie bestimmt werden sollen. Im Ablauf des Verfahrens muss die Videoerfassung der kompletten Schweinehälfte erfolgen und das Bildobjekt aufwändig bearbeitet sowie ausgewertet werden, um lediglich den Handelswert zu bestimmen. Auf Grund des großen Bildbereichs über die gesamte Spaltebene werden die Bewertungsgeschwindigkeit negativ beeinflusst als auch die Gewichte der Teilstücke nicht genau genug bestimmt. Andere derartige Verfahren zur nicht invasiven Bestimmung des Handelswerts und der Qualität von Schlachttierkörpern sind aus den Dokumenten DE 10050836 und DE 19952628 bekannt.

Die Aufgabe der Erfindung ist es, ein mehrstufiges Verfahren zur nichtinvasiven Bestimmung der Handelsklasse, des Handelswerts, dem Marktwert und der Qualität eines Schlächttierkörpers auf der Basis optischer Bildverarbeitung zu entwickeln, das die Bedingungen der einschlägigen amtlichen Vorschriften und Verordnungen erfüllt sowie genau, schnell als auch kostengünstig arbeitet.

Die Aufgabe wird durch die im Patentanspruch 1 aufgeführten Merkmale gelöst. Bevorzugte Weiterbildungen ergeben sich aus den Unteransprüchen.

Die Grundzüge des mehrstufigen Verfahrens zur nichtinvasiven Bestimmung der Handelsklasse, des Handelswerts, dem Marktwert und der Qualität eines Schlachttierkörpers stellen zunächst die Gewinnung von Basisdaten eines Schlachttierkörpers im realen Schlachtbetrieb als Datenvolumen, mit anschließender Simulationsrechnung zur Schätzung der Einzelteilausbeuten unter Einbeziehung von Verhältnisdaten dar. Diese Verhältnisdaten werden mittels Korrellation von Masseanteilen der Einzelteilausbeuten im Ergebnis von Zerlegeversuchen und parallel dazu mit einem automatischen Einstufungsverfahren, das mit optischer Bildauswertung einer Aufnahme des gespaltenen Schlachttierkörpers in der Schinken- Lendenregion arbeitet, ermittelter charakteristischer Messwerte und Parameter, gewonnen.

Das gesamte Verfahren, mit dem die Qualität und Quantitäten des Schlachttierkörpers bestimmt werden, besteht im Wesentlichen aus drei, sich in den Aussagen steigernden Stufen, wobei jedoch die Ergebnisse der einzelnen Stufen unabhängig von den anderen aus den Daten der optischen Bildauswertung im Aufnahmebereich ermittelt und ausgewiesen werden können.

Die Durchführung von Zerlegeversuchen einer Anzahl von Schlachtkörpern, hier Schweineschlachtkörpern bzw. deren Hälften, ist in europäischen und nationalen Verordnungen für die Zulassung von Verfahren zur Handelsklasseneinstufung vorgeschrieben. Im Rahmen der Zerlegeversuche nach Standardverfahren erfolgt die Berechnung des Muskelfleischanteils aus dem Gewicht des Filet, dem Gewicht des Muskelfleischs (einschließlich Bindegewebe) von Schulter, Kotelettstange, Schinken und Bauch, dem Gewicht der zerlegten Teilstücke sowie dem Gewicht der restlichen Teilstücke. Diese Zerlegeversuche sind protokollarisch mit allen Einzelheiten zu belegen.

Mit der genauen Protokollierung sind alle relevanten Daten erfasst und liegen als Datenvolumen vor, das die Gewichtsanteile der schwankenden Einzelteilausbeuten nichthomogener Schlachttierkörper mit hoher statistischer Genauigkeit beinhaltet.

Die Genauigkeit eines zugelassenen automatischen Einstufungsverfahrens zur Schätzung des Muskelfleischgehalts der Schlachtkörper muss dabei zum Beispiel mindestens dem Genauigkeitsgrad entsprechen, welche bei den Zerlegungsversuchen von 120 Schlachtkörpern mittels einfacher Regressionsrechnung erreicht würde.

Als automatisches Einstufungsverfahren kann ein bekanntes Verfahren eingesetzt werden, das charakteristische Messwerte und Parameter ausschließlich in der Lenden- und Schinkenregion durch Selektion markanter Punkte mittels optischer Bildauswertung eines mit einem optischen Sensor von der Spaltseite einer Schlachtkörperhälfte aufgenommenen Bildes ermittelt. Diese charakteristischen Messwerte und Parameter, wie Strecken, Winkel und Flächen als auch die ebenfalls mit dem Bild vorliegenden Helligkeits- bzw. Farbinformationen werden mit den Ergebnisdaten der Gewichtsanteile aus den Einzelteilausbeuten der Zerlegeversuche korreliert und daraus Verhältnisdaten gewonnen, die zusammen mit den Ausgangsdaten für spätere Rekursionsrechnungen gespeichert werden.

Unter anderem werden dabei exakte Messwerte für das Speckmaß (S) und das Fleischmaß (F) ermittelt, wobei der Muskelfleischanteil (MF%) eines Schlachtschweins nach dem ZP-Verfahren in Deutschland mit der amtlichen Formel unmittelbar berechnet werden, somit die Einstufung in die Handelsklasse als erste Verfahrensstufe sofort erfolgen kann.

Die Einstufung von Schlachtschweinekörpern mit länderspezifischen Formeln erfolgt in analoger Weise.

Als wesentliche Basisdaten eines Schlachtkörpers werden im Schlacht- oder Verarbeitungsbetrieb dessen Gewicht aus der Summe der an Haken hängenden, durch Teilung entlang der Wirbelsäule erhaltenen, Hälften bestimmt sowie die charakteristischen Messwerte und Parameter in der Lenden- und Schinkenregion durch Selektion markanter Punkte mittels optischer Bildauswertung eines mit einem optischen Sensor von der Spaltseite einer Schlachtkörperhälfte aufgenommenen digitalen oder digitalisierten Bildes ermittelt. An Hand der markanten Punkte erfolgt die Bestimmung charakteristischer Werte, Strecken, Winkeln und Flächen im Aufnahmebereich. Auf Basis ermittelter Längen von senkrechten Teilstrecken im Bereich des geraden Abschnitts der Wirbelsäule im Aufnahmebereich zur Außenkontur sowie dem Speckverlauf sowie deren Verhältnis zueinander werden in der zweiten Verfahrensstufe die Koteletten bewertet.

Mit den weiteren vorliegenden charakteristischen Werten erfolgt zur Schätzung der Einzelteilausbeute die Simulationsrechnung mittels rekursiver Berechnung unter Einbeziehung der Verhältnisdaten aus den Zerlegeversuchen. Aus der Summe der dabei erhaltenen Teilstückbewertung als dritte Verfahrensstufe ergibt sich wie üblich der Handelswert. Ausgehend vom Gewicht des Schlachtkörpers erfolgt die Schätzung der Gewichte der Teilstücke, aus deren Summe sich wiederum der Marktwert ergibt.

In diesem Zusammenhang ist es ebenso denkbar, die Schätzung der Gewichte der Teilstücke lediglich auf Grundlage der im Aufnahmebereich ermittelten charakteristischen Werte, Strecken, Winkel und Flächen vorzunehmen, ohne zunächst das Gesamtgewicht des Schlachttierkörpers zu bestimmen und in deren Berechnung einzubeziehen.

Mit Hilfe der Helligkeits- bzw. Farbinformationen erfolgt die Qualitätseinstufung des Schlachtkörpers.

Die Vorteile der Erfindung bestehen insbesondere in der Möglichkeit des Einsatzes bekannter, nichtinvasiver, automatischer Verfahren zum Ermitteln von Messwerten zur Bestimmung des Muskelfleischanteils (MF%) von Schlachtschweinen nach amtlichen Vorschriften. Es sind sowohl bildgebende Verfahren nutzbar, die eine Aufnahme in der Spaltebene auswerten als auch Verfahren zur Vermessung des Schlachtkörpers entlang des Rückgrats durch Kernspinoder Computertomografie oder Ultraschall.

Die zulässigen Toleranzen für den, nach amtlichen Vorgaben zulässigen Schätzfehler für den Muskelfleischanteil werden eingehalten und sogar unterschritten.

Mit der Einschränkung des zur Bewertung benutzten Aufnahmebereichs auf die Schinken- und Lendenregion lassen sich exakte Messwerte bestimmen, wodurch genauere Auswertungen bei höherer Geschwindigkeit ergeben.

Der Handelswert des Schlachtkörpers kann aus den Daten für die werttragenden Teilstücke ermittelt werden. Unter Berücksichtigung der gesamten Masse lässt sich der Marktwert berechnen.

Mit dem beschriebenen Verfahren sind bisher bekannte Insellösungen zur Bestimmung des Muskelfleischanteils und gegebenenfalls des Handelswertes ersetzbar, um genau, schnell und kostengünstig alle Parameter für die Verarbeitung, Weiterverarbeitung sowie Vergütung bestimmen zu können.

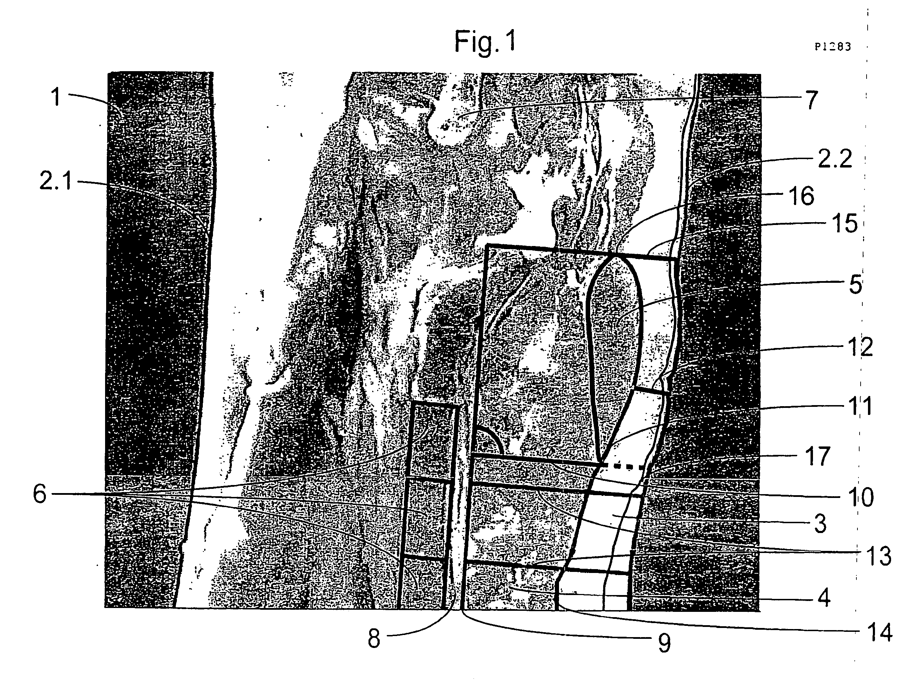

Die Erfindung wird als Ausführungsbeispiel an Hand von Fig. 1 als Aufnahmebereich für die Bestimmung charakteristischer Messwerte und Parameter an einer Hälfte eines Schlachtkörpers näher erläutert.

Bei Zerlegeversuchen einer genügenden Anzahl von Schweineschlachtkörpern werden zur Gewinnung von Basisdaten zunächst deren Gewicht nach dem Stechen und Abkühlen bestimmt, wobei die Schlachtkörper bereits entlang der Wirbelsäule geteilt sein können, danach in der Lenden- und Schinkenregion mit einem bildgebenden Verfahren ein digitales Bild erstellt, das einer Bildanalyse unterzogen wird, bei der Konturverläufe von Fleischgewebe und Fettgewebe und Knochen erfasst werden. Anhand der Konturverläufe werden einzelne Strecken, über Kontur-bereiche gemittelte Strecken und Flächen gemessen als auch Helligkeits- und/oder Farbwerte gewonnen. Nachfolgend erfolgt der eigentliche Zerlegeversuch, der exakt zu protokollieren ist, bei dem die Gewichtsanteile aller Teilstücke einzeln bestimmt und gespeichert werden.

Den mit automatischer Bildanalyse gewonnenen Messwerten und Parametern werden jeweils das Gewicht des Schlachtkörpers und die Gewichte der Einzelteilausbeuten zugeordnet, aus denen die Berechnung spezifischer Verhältnisdaten erfolgt. Auf Grund des umfangreichen Datenvolumens aus einer Vielzahl von Zerlegeversuchen sind diese Verhältnisdaten statistisch gesichert.

Die Gewinnung der charakteristischen Messwerte und Parameter in der Schinken- und Lendenregion bei Zerlegeversuchen als auch im laufenden Schlachtbetrieb erfolgt mit der jeweils gleichen Methode, vorzugsweise in Anlehnung an das in der Druckschrift DE 199 36 032 C1 beschriebene Verfahren. Nach Fig.1 wird hierbei ein Bildbereich 1 der Schinken- und Lendenregion einer Schlachtkörperhälfte mit allen Details aufgenommen sowie photogrammetrisch ausgewertet.

Der Bildbereich 1 erfasst im Kontrast zu einem dunklen Hintergrund die gesamte Breite der Schinken- und Lendenregion mit deren Außenkonturen 2.1 und 2.2.

Anhand einer Histogrammanalyse erfolgt zunächst die Renormierung der Schwellenparameter auf die jeweilige Helligkeit des Schweineschlachtkörpers mit anschließender rechentechnischer Selektion der unterschiedlichen Gewebepartien auf Basis der Farb- und/oder Helligkeitsunterschiede im Bildbereich 1. In üblicher Weise werden mit Hilfe von Selbstkonsistenzprüfungen Verunreinigungen, wie durch Blut, aus dem Bild herausgefiltert.

Im nächsten Schritt wird der helle Speck vom dunkleren Fleisch separiert und auf diese Weise Speckflächen 3 und Fleischflächen 4 bestimmt. Innerhalb der Fleischflächen 4 wird die Kontur eines Musculus Glutaeus Medius (MGM) 5 durch einen Konturverfolgungsalgorithmus mit anschließender Bestimmung der geometrischen Lage identifiziert. Des Weiteren sind im Bildbereich 1 das untere Ende der Wirbelsäule mit Wirbeln 6 sowie ein Schlossknochen 7 sichtbar. Die Wirbel 6 mit Wirbelkanal 8 im geraden Teil der Wirbelsäule werden hierbei anhand von Periodizitätskriterien ermittelt.

An die obere (dorsale) Kante des Wirbelkanals 8 wird eine Gerade 9 mit der Richtung des geraden Abschnitts der Wirbelsäule als Ausgangslinie für die Messungen gelegt. Auf dieser Geraden 9 wird eine Senkrechte 10 in Höhe eines vorderen (cranialen) Ende 11 des MGM 5 errichtet, deren Strekkenlänge als kürzeste Verbindung vom vorderen Ende 11 des MGM 5 zur oberen (dorsalen) Kante des Wirbelkanals 8 dem Fleischmaß (F) als Stärke des Lendenmuskels entspricht. Die Verlängerung der Senkrechten 10 bis zur Außenkontur 2.2 begrenzt den Speckverlauf über dem MGM 5 cranial.

In Höhe der dünnsten Speckschicht am MGM 5 wird eine Verbindungslinie 12 von der Kontur des MGM 5 zur Außenkontur 2.2. bestimmt, deren Streckenlänge das Speckmaß (S) repräsentiert.

Aus den beiden Termen (F) und (S), gemessen in Millimetern, erfolgt online die Berechnung des Muskelfleischanteils (MF%) nach dem ZP-Verfahren mit der spezifischen amtlichen Formel mit nachfolgender Handelsklasseneinstufung auf Grundlage des ermittelten Muskelfleischanteils.

Parallel zur Senkrechten 10 können weitere senkrechte Strecken 13 auf der Geraden 9 zur Außenkontur 2.2 berechnet werden, deren Ausgangspunkt auf der Geraden 9 jeweils in der virtuellen senkrechten Verlängerung der Schicht zwischen den Wirbeln 6 liegt. Die senkrechten Strecken 13 werden von einer inneren Konturlinie 14 der Speckfläche 3 geschnitten, so dass Teilstrecken im Muskelfleisch sowie dem Speck entstehen, aus deren Längen als Speck- und Muskelstrecken sowie dem Verhältnis dieser zueinander eine Bewertung der Koteletten erfolgt.

Zum Bewerten des Schinken wird die mittlere Speckdicke über dem MGM 5, im Bereich der Fläche zwischen der Verlängerung der Senkrechten 10 bis zur Außenkontur 2.2 und einer anderen Senkrechten 15 auf der Geraden 9 in Höhe eines hinteren (caudalen) Ende 16 des MGM 5 einbezogen, die ebenfalls in die Handelswertbestimmung eingeht.

Im Bildbereich 1 werden eine Anzahl weiterer, über das beschriebene Beispiel hinausgehende, Strecken, Winkel und Flächen bestimmt, die zur Verfeinerung der Differenzierung der Verhältnisdaten beitragen.

So werden Aussagen zum Bauch anhand einer mittleren Unterhautfettdicke 17 im Kotelettbereich, im Bildbereich 1 ab dem cranialen Ende 11 des MGM 5 und der Schulter anhand Schinken, Kotelett und Bauch aus den weiteren Messwerten getroffen.

Mit den aus der Bildanalyse zuvor gewonnenen Daten werden unter Einbeziehung des erfassten Gesamtgewichts des Schlachtkörpers aus den beiden am Haken hängenden, zusammengehörenden Hälften, auf Basis der im Datenvolumen vorliegenden Verhältnisdaten die Einzelteilausbeuten berechnet, womit sich aus der Summe der Teilstückbewertungen der Handelswert sowie aus der Summe der Gewichte der Teilstücke der Marktwert ergibt.

Es ist vorstellbar, das Gewicht von Teilstücken, wie dem Schinken oder den Koteletten direkt aus den Messwerten der Bildanalyse zu bestimmen.

Des Weiteren erfolgt die Qualitätseinstufung des Schlachtkörpers und/oder von Teilstücken an Hand der vorliegenden Helligkeits- und/oder Farbwerte.

Eine Weiterentwicklung des Verfahrens, das insbesondere in Zerlegebetrieben zum Einsatz kommen kann, weist einen implementierten Selbstlerneffekt mit Selbstkonsistenzprüfung des Datenvolumens auf, indem die Ergebnisse der im Verarbeitungsprozess erfolgenden Wägungen von Teilstücken mit den im Datenvolumen vorhandenen Werten abgeglichen, gegebenenfalls mit weiteren Daten ergänzt werden, wodurch insbesondere die Varianz der Schätzergebnisse für die Teilstückausbeuten weiter eingeschränkt wird.

Auf diese Weise gewonnene, erweiterte Datenvolumina werden als Upgrade in kleinen Schlachtbetrieben genutzt, um dort ebenfalls noch genauere Schätzergebnisse zu erzielen.

Sämtliche Verfahrensschritte erfolgen mit der Nutzung elektronischer Datenverarbeitungseinrichtungen, die unter anderem geeignete Schnittstellen zu bildgebenden Einrichtungen und Wägeeinrichtungen aufweisen.

Verwendete Bezugszeichen

1        Bildbereich

| 2 | Außenkontur (2.1; 2.2) |
|---|---|
| 3 | Speckflächen |
| 4 | Fleischflächen |
| 5 | Musculus Glutaeus Medius (MGM) |
| 6 | Wirbel |
| 7 | Schloßknochen |
| 8 | Wirbelkanal |
| 9 | Gerade |
| 10 | Senkrechte |
| 11 | vorderes (craniales) Ende |
| 12 | Verbindungslinie |
| 13 | weitere senkrechte Strecken |
| 14 | innere Konturlinie |
| 15 | andere Senkrechte |
| 16 | hinteres (caudales) Ende |
| 17 | mittlere Unterhautfettdicke |

**Patentansprüche**

1. Verfahren zur nichtinvasiven Bestimmung der Handelsklasse, des Handelswerts, dem Marktwert und der Qualität eines Schlachttierkörpers auf der Basis optischer Bildverarbeitung, bei dem im Bildbereich (1) in der Schinken- und Lendenregion mit allen Details, Strecken, Winkel, Flächen, Helligkeits- und/oder Farbinformationen bestimmt werden, das Gesamtgewicht eines Schlachtkörpers sowie Daten von Ergebnissen aus Zerlegeversuchen zu schwankenden Einzelteilausbeuten nichthomogener Schlachttierkörper erfasst und einbezogen werden, wobei die mit Zerlegeversuchen einer genügenden-Anzahl von Schlachtkörpern gewonnenen Ergebnisdaten von Gewichtsanteilen aus Einzelteilausbeuten mit den von beiden Hälften eines Schlachtkörpers in der Schinken- und Lendenregion ermittelten charakteristischen Messwerten und Parameter miteinander unter optionaler Einbeziehung des Gesamtgewichts korreliert und daraus Verhältnisdaten gewonnen werden und im laufenden Schlachtbetrieb zur Schätzung der Einzelteilausbeuten eine Simulationsrechnung mit den vorliegenden Verhältnisdaten eines Schlachtkörpers, und der für diesen spezifisch in der Schinken- und Lendenregion ermittelten charakteristischen Messwerte und Parameter erfolgt, **dadurch gekennzeichnet, dass** parallel zu einer Senkrechten (10),in Höhe eines vorderen (cranialen) Endes (11) eines Musculus Glutaeus Medius - MGM (5), die in einem Teilschritt der Bildauswertung zur Online-Berechnung des Muskelfleischanteils (MF%) auf einer an die obere (dorsale) Kante eines Wirbelkanals (8) mit der Richtung des geraden Abschnitts der Wirbelsäule gelegten Geraden (9) errichtet wurde, weitere senkrechte Strecken (13) auf der Geraden (9) zur Außenkontur (2.2) berechnet werden, deren Ausgangspunkte auf der Geraden (9) jeweils in der virtuellen senkrechten Verlängerung der Schicht zwischen den Wirbeln (6) liegen, wobei die senkrechten Strekken (13) von einer inneren Konturlinie (14) einer Speckfläche (3) geschnitten werden, so dass Teilstrecken im Muskelfleisch sowie dem Speck entstehen, deren Längen als Speck- und Muskelstrecken sowie das Verhältnis dieser zueinander zur Bewertung der Koteletten benutzt werden.

2. Verfahren nach Anspruch 1, **dadurch gekennzeichnet,**

**dass** im laufenden Schlachtbetrieb zur Schätzung der Einzelteilausbeuten eine Simulationsrechnung mit den vorliegenden Verhältnisdaten eines Schlachtkörpers unter Berücksichtigung des Gesamtgewichts, und der für diesen spezifisch in der Schinken- und Lendenregion ermittelten charakteristischen Messwerte und Parameter erfolgt.

3. Verfahren nach Anspruch 1 oder 2, **dadurch gekennzeichnet,**
**dass** in Höhe der dünnsten Speckschicht am MGM (5) eine Verbindungslinie (12) von der Kontur des MGM (5) zur Außenkontur (2.2) bestimmt wird, deren Streckenlänge das Speckmaß (S) repräsentiert, und dem Fleischmaß (F), als Stärke des Lendenmuskels, das der Streckenlänge der in Höhe des vorderen (cranialen) Endes (11) des Musculus Glutaeus Medius - MGM (5) errichteten Senkrechten (10), als kürzeste Verbindung vom vorderen Ende (11) des MGM (5) zur oberen (dorsalen) Kante des Wirbelkanals (8), entspricht, online aus den beiden Termen (F) und (S) die Berechnung des Muskelfleischanteils (MF%) nach dem ZP-Verfahren mit der spezifischen amtlichen Formel mit nachfolgender Handelsklasseneinstufung erfolgt.

4. Verfahren nach den Ansprüchen 1 oder 2 und 3, **dadurch gekennzeichnet,**
**dass** das Gewicht von Teilstücken, wie dem Schinken oder den Koteletten, direkt aus den Messwerten der Bildanalyse bestimmt wird.

5. Verfahren nach den Ansprüchen 1 oder 2 und 3 bis 4, **dadurch gekennzeichnet,**
**dass** die mittlere Speckdicke über dem MGM (5), im Bereich der Fläche zwischen der Verlängerung der Senkrechten (10) bis zur Außenkontur (2.2) und einer anderen Senkrechten (15) auf der Geraden (9) in Höhe eines hinteren (caudalen) Ende (16) des MGM (5) zum Bewerten des Schinken einbezogen wird und in die Handelswertbestimmung eingeht.

6. Verfahren nach den Ansprüchen 1 oder 2 und 3 bis 5, **dadurch gekennzeichnet,**
**dass** Aussagen zum Bauch anhand einer mittleren Unterhautfettdicke (17) im Kotelettbereich, im Bildbereich (1) ab dem cranialen Ende (11) des MGM (5) und der Schulter anhand Schinken, Kotelett und Bauch aus den weiteren Messwerten getroffen werden.

7. Verfahren nach den Ansprüchen 1 oder 2 und 3 bis 6, **dadurch gekennzeichnet,**
**dass** das Verfahren bei Einsatz in Zerlegebetrieben einen implementierten Selbstlerneffekt mit Selbstkonsistenzprüfung des Datenvolumens aufweist, wobei die Ergebnisse der im Verarbeitungsprozess erfolgenden Wägungen der Teilstücke mit den im Datenvolumen vorhandenen Werten abgeglichen und gegebenenfalls mit weiteren Daten ergänzt werden.

8. Verfahren nach den Ansprüchen 1 oder 2 und 3 bis 7, **dadurch gekennzeichnet,**
**dass** durch den Selbstlerneffekt erweiterte Datenvolumina als Upgrade in kleinen Schlachtbetrieben genutzt werden.

**Claims**

1. Method of determining in a non-invasive manner the trade classification, the trade value, the market value and the quality of a body of a slaughtered animal on the basis of optical image processing, wherein in the image region (1) in the ham and loin region lengths, angles, areas, brightness and/or colour information are determined with all details, the total weight of a carcass and data from results of tests on cut pieces with respect to fluctuating yields of individual pieces of non-homogenous bodies of slaughtered animals are recorded and used, wherein the result data, obtained with tests on cut pieces of a sufficient number of carcasses, of weight percentages from yields of individual pieces with the characteristic measured values and parameters determined from two halves of a carcass in the ham and loin region are correlated together optionally using the total weight and relational data are obtained therefrom and in the active slaughtering operation in order to estimate the yields of individual pieces a simulation calculation is performed with the available relational data of a carcass and the characteristic measured values and parameters determined for this specifically in the ham and loin region, **characterised in that**
in parallel with a perpendicular (10) at the level of a front (cranial) end (11) of a musculus glutaeus medius - MGM (5), which was created in a part step of the image evaluation in order to perform an online calculation of the muscle-meat percentage (MF %) on a straight line (9) placed at the upper (dorsal) edge of a vertebral canal (8) in the direction of the straight section of the spinal column, further perpendicular lengths (13) are calculated on the straight line (9) to the outer contour (2.2), the starting points of which lengths on the straight line (9) lie in each case in the virtual perpendicular extension of the layer between the vertebrae (6), wherein the perpendicular lengths (13) are cut from an inner contour line (14) of a fat area (3), so that part lengths are created in the muscle meat and the fat, whose lengths as fat and muscle lengths and the relationship of these with respect to each other are used to evaluate the cutlets.

2. Method as claimed in Claim 1, **characterised in that** in the active slaughtering operation in order to estimate the yields of individual pieces a simulation calculation is performed with the available relational data of a carcass taking into consideration the total weight and the characteristic measured values and parameters determined for this specifically in the ham and loin region.

3. Method as claimed in Claim 1 or 2, **characterised in that** at the level of the thinnest fat layer at the MGM (5) a connection line (12) from the contour of the MGM (5) to the outer contour (2.2) is determined, the extension length of this connection line representing the amount of fat (S) and the meat measurement (F) as the thickness of the loin muscle, which corresponds to the extension length of the perpendicular (10) created at the level of the front (cranial) end (11) of the musculus glutaeus medius - MGM (5) as the shortest connection from the front end (11) of the MGM (5) to the upper (dorsal) edge of the vertebrae channel (8), the muscle-meat percentage (MF %) is calculated online from the two terms (F) and (S) in accordance with the two-point method using the specific official formula and subsequent classification into the trade class.

4. Method as claimed in Claims 1 or 2 and 3, **characterised in that** the weight of pieces, such as the ham or the cutlets, is determined directly from the measured values of the image analysis.

5. Method as claimed in Claims 1 or 2 and 3 to 4, **characterised in that** the average fat thickness over the MGM (5) in the region of the area between the extension of the perpendiculars (10) as far as the outer contour (2.2) and another perpendicular (15) on the straight line (9) at the level of a rear (caudal) end (16) of the MGM (5) is used to evaluate the ham and is included in the determination of the trade value.

6. Method as claimed in Claims 1 or 2 and 3 to 5, **characterised in that** statements regarding the belly using a mean panniculus adiposus thickness (17) in the cutlet region are provided in the image region (1) from the cranial end (11) of the MGM (5) and the shoulder using ham, cutlet and belly from the further measured values.

7. Method as claimed in Claims 1 or 2 and 3 to 6, **characterised in that** the method when used in butchering operations comprises an implemented self-learning effect with self-consistency checks on the data volume, wherein the results of the weighing of pieces performed during processing are aligned with the values provided in the data volume and are supplemented if necessary with other data.

8. Method as claimed in Claims 1 or 2 and 3 to 7, **characterised in that** data volumes expanded by virtue of the self-learning effect are used as an upgrade in small slaughtering operations.

**Revendications**

1. Procédé de détermination non invasive de la classe commerciale, de la valeur commerciale, de la valeur de marché et de la qualité d'une carcasse d'animal abattu sur la base d'un traitement optique d'images, selon lequel dans la zone de l'image (1) on détermine pour la région des jambons et la région lombaire, avec tous les détails, des lignes, angles, surfaces et informations de luminosité et/ou de couleur, et on saisit et prend en compte le poids total d'une carcasse ainsi que des données résultant d'essais de découpage destinés à donner des rendements de parties individuelles, fluctuants, de carcasses non homogènes, les données concernant les fractions pondérales déterminées à partir des rendements de parties individuelles résultant d'un nombre suffisant d'essais de découpage de carcasses, étant corrélées avec les valeurs mesurées et les paramètres caractéristiques déterminés pour les deux moitiés d'une carcasse, dans la région des jambons et dans la région lombaire, en prenant optionnellement en compte le poids total, et des données de rapport en étant déduites, et dans un abattoir en service, pour déterminer les rendements de parties individuelles un calcul de simulation étant effectué en utilisant les données de rapport existantes d'une carcasse, et les valeurs de mesure et paramètres caractéristiques établis pour cette carcasse spécifiquement dans la région des jambons et dans la région lombaire, **caractérisé en ce que** parallèlement à une perpendiculaire (10), à la hauteur d'une extrémité avant (crânienne) d'un musculus glutaeus medius MGM (5), qui a été dressée dans une étape partielle d'exploitation d'images destinée au calcul en direct de la fraction de viande de muscle (MF%) sur une droite (9) dirigée sur le bord supérieur (dorsal) d'un canal rachidien (8), selon la partie droite rectiligne de la colonne vertébrale, on calcule d'autres parcours (13) perpendiculaires, à la droite (9), aboutissant au contour externe (2.2), avec chacun comme point de départ sur la droite (9) un point situé sur le prolongement vertical virtuel de la couche située entre les vertèbres (6), les parcours (13) perpendiculaires étant coupés par une ligne de contour interne (14) d'une surface de lard (3), de sorte que dans la viande de muscle et également dans le lard se trouvent des parcours partiels dont les longueurs et leur rapport sont utilisés pour évaluer les côtelettes.

2. Procédé selon la revendication 1,
   **caractérisé en ce que**
   pendant le fonctionnement courant de l'abattoir, pour déterminer les rendements de parties individuelles on fait un calcul de simulation utilisant les données de rapport existantes d'une carcasse, en tenant compte du poids total, ainsi que les valeurs de mesure et paramètres caractéristiques établis pour cette carcasse spécifiquement dans la région des jambons et dans la région lombaire.

3. Procédé selon la revendication 1 ou 2,
   **caractérisé en ce que**
   à la hauteur de la couche de lard la plus faible sur le MGM (5) on définit une ligne de liaison (12) qui va du contour du MGM (5) au contour extérieur (2.2), et dont la longueur représente la masse de lard (S), et la longueur de la perpendiculaire (10) dressée à la hauteur d'une extrémité avant (crânienne) du musculus glutaeus medius MGM (5) correspond, en tant que liaison la plus courte entre l'extrémité avant (11) du MGM (5) et le bord supérieur (dorsal) du canal rachidien (8) à la masse de viande (F) en tant qu'épaisseur du muscle lombaire, et à partir des deux termes (F, S) on effectue le calcul direct de la fraction de viande de muscle (MF%), selon le procédé (ZP) en utilisant la formule officielle spécifique ainsi que la classification périodique qui en résulte.

4. Procédé selon les revendications 1 ou 2 et 3,
   **caractérisé en ce que**
   le poids de morceaux individuels, tels que les jambons ou les côtelettes, est déterminé directement à partir des valeurs de mesure données par l'analyse de l'image.

5. Procédé selon les revendications 1 ou 2 et 3 à 4,
   **caractérisé en ce que**
   l'épaisseur moyenne de lard sur le MGM (5) dans la zone de la surface comprise entre le prolongement de la perpendiculaire (10) allant jusqu'au contour externe (2.2) et une autre perpendiculaire (15) à la droite (9) à la hauteur d'une extrémité arrière (caudale) (16) de MGM (5), est prise en compte pour évaluer le lard et utilisée dans la détermination de la valeur commerciale.

6. Procédé selon les revendications 1 ou 2 et 3 à 5,
   **caractérisé en ce que**
   des informations concernant l'abdomen sont obtenues au moyen d'une épaisseur moyenne de graisse sous-cutanée (17) dans la zone des côtelettes, dans la zone d'image (1) à partir de l'extrémité crânienne (11) de MGM (5) et de l'épaule, à l'aide des jambons, des côtelettes et de l'abdomen à partir des autres valeurs de mesure.

7. Procédé selon les revendications 1 ou 2 et 3 à 6,
   **caractérisé en ce que**
   le procédé, quand il est utilisé dans des abattoirs, présente un effet auto-adaptatif implémenté avec contrôle automatique de consistance du volume des données, les résultats des pesées des parties individuelles effectuées pendant le processus de traitement étant comparés aux valeurs existant dans le volume des données et complétés éventuellement par d'autres données.

8. Procédé selon les revendications 1 ou 2 et 3 à 7,
   **caractérisé en ce que**
   les volumes de données, amplifiés par l'effet auto-adaptatif sont utilisés comme élément de revalorisation dans de petits abattoirs.

Fig.1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DD 298310 A5
- DE 4131556 C2
- DE 4109345 C2
- DE 19733216 C1
- DE 19847232 C2
- DE 19936032 C1
- DE 19952628 A1
- DE 10050836
- DE 19952628